# EUROPEAN PATENT SPECIFICATION

(11) **EP 0 420 486 B1**
(45) Date of publication and mention of the grant of the patent: **20.04.1994**
(21) Application number: 90310238.2
(22) Date of filing: 19.09.1990
(51) Int. Cl.: A61M 29/02, A61M 25/10

(54) **Small diameter dilatation catheter having wire reinforced coaxial tubular body**
Dilatationskatheter mit geringem Durchmesser, ausgestattet mit einem drahtverstärkten, koaxialen, schlauchförmigen Körper
Cathéter de dilatation à petit diamètre comprenant un corps tubulaire coaxial renforcé par fils

(30) Priority: 25.09.1989 US 411815
(43) Date of publication of application: 03.04.1991
(73) Proprietor: SCHNEIDER (USA) INC., Plymouth, Minnesota 55442 (US)
(72) Inventor: Aase, Brenda L., Eagan, Minnesota (US)
(74) Representative: Bradbrook, Geoffrey William

(56) References cited:
- EP-A- 0 318 918
- EP-A- 0 349 640
- GB-A- 1 566 674

## Description

This invention relates generally to dilatation catheters for use in carrying out percutaneous transluminal coronary angioplasty (PTCA) procedures and more specifically to such a catheter which is designed to have a catheter body of very low diameter while still possessing the requisite firmness allowing it to be advanced through the vascular system without kinking or accordion pleating.

The PTCA procedure for restoring patency to occluded or partially occluded coronary arteries is generally attributed to A. Gruntzig. As is now well known, the procedure involves inserting an elongated tubular catheter having a balloon or expander member on its distal end into the vascular system and then advancing the catheter until the balloon spans or bridges the stenosis to be treated. Then, an inflation fluid is injected through the distal end of the catheter and made to perfuse along the length thereof to inflate the expander member to a predetermined size and pressure.

Since the procedure was first introduced, considerable work has gone on in developing improved catheters for carrying but this procedure. Much of the effort on the part of the various catheter manufacturing companies has been in attempting to reduce the overall diameter of the catheter so that it may more readily be passed through small diameter coronary blood vessels. When it is also considered that a PTCA catheter must be used in combination with a guide wire, which extends completely through the length of the catheter, the catheter shaft requires two lumens. The two lumens may be created by extruding or otherwise forming a double lumen tube or, alternatively, by providing two concentrically disposed tubes where the lumen of the centermost tube accommodates the guide wire and the passage between the O.D. of the inner tube and the I.D. of the outer tube allows for the perfusion of inflation fluid to the balloon.

The catheter of WO-A-8 806 465, which is regarded to be the closest prior art, is of greater diameter (inner tube 0.4-2.5mm, see page 13 last line) and the reinforcing member 13 shown in Fig. 14 is wound around the inner tube to prevent flexion of the catheter and improve torque transmission. Such an arrangement is not suitable for the very thin-walled catheters of this invention and is far less effective in preventing the concertina effect as the wound or meshed wire can contract in the manner of a coil spring.

In striving for low catheter shaft diameters, the wall thickness of the tubes becomes a critical factor. If made overly thin, the resulting catheter lacks sufficient longitudinal rigidity and tends to fold upon itself when an effort is made to push the PTCA catheter through its guide catheter. Thus, a practical limit exists on the minimum wall thickness while still permitting the necessary "pushability" characteristic necessary for advancing the catheter through the vascular system.

It is accordingly a principal object of the present invention to provide an improved PTCA catheter whose tubular body dimension is substantially smaller than any known prior art catheter for the same purpose presently on the market.

Another object of the invention is to provide a small diameter, thin-wall PTCA balloon catheter which exhibits sufficient longitudinal rigidity to allow it is be passed through the vascular system without kinking or pleating.

A further object of the invention is to provide a balloon catheter for use in PTCA procedures of the type including two coaxially disposed tubes of thin wall dimension, yet appropriately reinforced against accordion pleating when the catheter assembly is pushed through a guide catheter and into a blood vessel to be treated.

The foregoing features and advantages of the invention are achieved by providing first and second elongated flexible tubular members each of minimal wall thickness, the tubular members preferably being formed from a suitable polymer such as polyamide, polyester or polyimide with a polyimide plastic being preferred, and incorporating a reinforcing structure within the walls of one or both tubes. The tubes are coaxially disposed relative to one another with the smaller diameter tube having its distal end projecting beyond the distal end of the larger diameter tube. The inflatable expander member is circumferentially bonded at its proximal end about the distal end portion of the larger diameter tube and its distal end is circumferentially bonded to the distal end portion of the smaller diameter, concentrically disposed tube.

A molded plastic hub is affixed to the proximal ends of the two tubes and includes an inflation port in fluid communication with the annular space existing between the O.D. of the small diameter tube and the I.D. of the larger diameter tube. Inflation fluid injected through the inflation port then perfuses through this space to fill and inflate the expander member to a desired pressure. The lumen of the inner tube can accommodate a guide wire.

In accordance with a first embodiment of the invention, the reinforcing structure may comprise a single strand of wire extending substantially the entire length of the catheter and embedded within the wall of the larger diameter tube, the smaller diameter tube, or both. In another arrangement, plural strands of wire are incorporated in the wall of the tubes, running parallel to one another.

The constructional features of the catheter of the present invention can be better perceived from the following detailed description of the preferred embodiments thereof, especially when considered in conjunction with the accompanying drawings in which:
Figure 1 is a partially sectioned side elevation of a PCTA catheter constructed in accordance with the present invention;
Figure 2 is a cross-sectional view of a catheter like that of Figure 1 showing an alternative reinforcing structure;
Figure 3 is another cross-sectional view illustrating yet another way of providing longitudinal reinforcement; and
Figure 4 is yet another cross-sectional view of a catheter like that of Figure 1 except incorporating a still difference reinforcing structure.

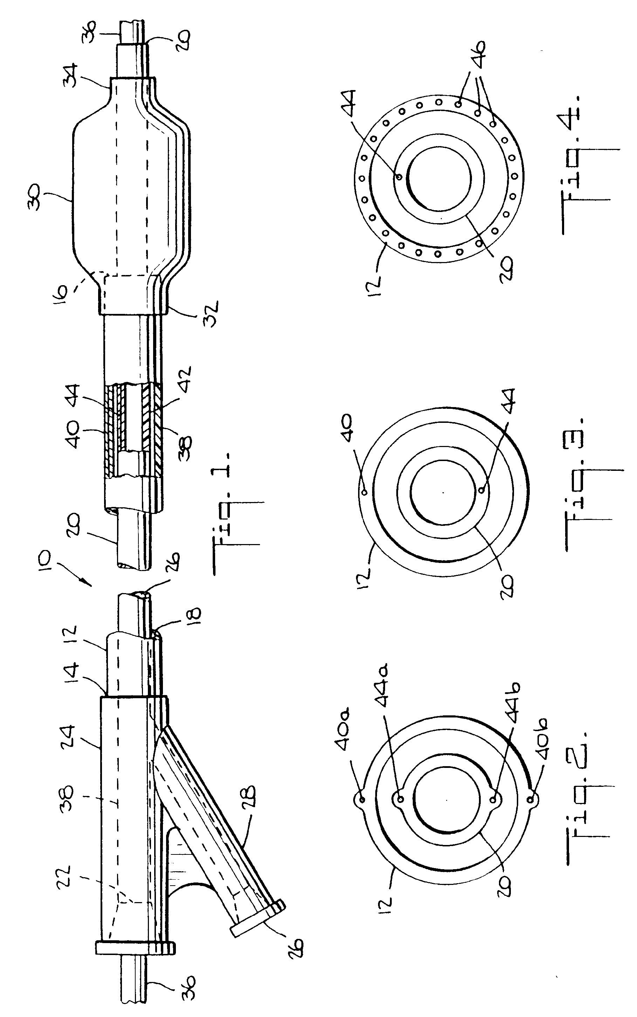

Referring first to Figure 1, the PCTA catheter constructed in accordance with the present invention is indicated generally by numeral 10. The catheter includes an outer tubular body member 12 having a proximal end 14 and a distal end 16 with a lumen 18 extending from the proximal end to the distal end.

Coaxially disposed within the lumen 18 of the outer tubular member 12 is an inner tubular member 20 having a proximal end 22 and a distal end 24 with a lumen 26 extending the full length thereof.

Affixed to the proximal end 14 of the outer tubular member 12 and the proximal end 22 of the inner tubular member 20 is a molded plastic hub 24 which is commonly referred to as a Y-connector. The connector includes a Luer fitting 26 at the end of a longitudinal bore passing through the arm 28 and in fluid communication with the annular space existing between the O.D. of the inner tube 20 and the I.D. of the outer tube 12.

Affixed to the distal end portion of the catheter 10 is an inflatable expander member 30. The expander member is also tubular in form and includes a proximal end 32 which is appropriately circumferentially bonded to the exterior wall surface of the outer tubular member 12. The distal end 34 of the expander member 30 is likewise appropriately bonded to the exterior wall surface of the inner tubular member 20 which extends outwardly beyond the distal end 16 of the outer tubular member. It can be seen, then, that when an inflation fluid is injected under pressure through the port 26, it will perfuse through the annular space between the two tubular members and will exit the distal end of the outer tubular member 12 into the interior of the inflatable expander member 30.

As is well known in the art, the expander member 30 may comprise a tubular film of polyethylene terephthalate (PET) which is biaxially oriented in a drawing and blow molding process so as to exhibit a very high burst strength well in excess of 7,03 kg/cm² (100 PSI) and which does not appreciably expand radially beyond a predetermined maximum diameter even with substantially increased pressures.

With continued reference to Figure 1, there is also shown a guide wire 36 which passes through a bore 38 formed in the hub 24 and through the lumen 26 of the inner tube 20 so as to exit the distal end 24 thereof. Guide wires are commonly used in PTCA procedures for facilitating the steering of the catheter 10 through the vascular system to a location where the expander member 30 is disposed adjacent a stenotic lesion to be treated.

Referring to the sectioned portion of the catheter assembly 10 of Figure 1, it will be noted that there is disposed within the wall thickness 38 of the outer tubular member 12 a reinforcing member 40 which, in Figure 1, comprises a fine strand of wire, preferably stainless steel. The wall 42 of the inner tube 20 also is seen to include a stiffening wire 44. In practice, a workable PTCA catheter system may result when a reinforcing strand is incorporated in only one of the two concentric tubes. The diameter of the reinforcing strand is 0.025 mm (0.001 inch) to 0.05 mm (0.002 inch).

In fabricating the tubes 12 and 20, a solid cylindrical mandrel approximately five feet long may first be coated with multiple think layers of plastic. While various plastics including polyesters, polyamides and polyimides may be employed, it has been found that polyimide is preferred because of its characteristics of tensile strength and flexibility.

Once the mandrel is so coated, the reinforcing wire(s) 40 or 44 may be positioned on the coating covering the mandrel and then that assembly is repeatedly dipped, sprayed or otherwise coated with additional layers of the same plastic whereby the wire reinforcing strand becomes totally embedded within the tube's wall.

Figure 2 illustrates how a plurality of strands 40a and 40b can be embedded in the wall of the outer tubular member 12 and, likewise, how plural reinforcing strands 44a and 44b can be embedded in the wall of the inner tubular member 20. In figure 2, the strands are shown as being encased in ribs projecting from the exterior walls of the tubes. In the view of Figure 3, however, the wire reinforcing members 40 and 44 are wholly contained within the walls of the tubes 12 and 20.

The cross-sectional view of Figure 4 shows a further alternative arrangement. Here, the inner tubular member 20 is seen to include a single reinforcing strand 44 while the outer tubular member 12 incorporates a plurality of wire strands in the form of a loosely woven braid 46.

Using the approach of the present invention, it has been found possible to construct a coaxial PTCA catheter whose outer tube 12 may have an O.D. as small as 0.75 mm (0.030 inch) with a wall thickness of about 0.025 mm (.001 inch) leaving a lumen of about 0.71 mm (.028 inch) in diameter. A lumen of this size can accommodate an inner tube 20 having a O.D. in the range of from 0.47 mm (.0185 inch) to 0.53 mm (.021 inch) and a corresponding I.D. of from 0.43 mm (.017 inch) to 0.475 mm (.0187 inch). The lumen of the inner tube can then accommodate a guide wire 36 whose O.D. may be typically 0.35 mm (.014 inch).

Those skilled in the art will appreciate that the ongoing dimensions are exemplary only and are included to illustrate the fact that by choosing an appropriate plastic material (polyimide) and by incorporating a fine wire strand in the wall of the tube(s) comprising the catheter, a coaxial catheter can be constructed which will have a very low overall outer dimension yet will possess the necessary longitudinal rigidity to as to avoid the tube collapsing in the longitudinal direction and becoming accordion pleated when pushed or advanced through a guide catheter or otherwise through the vascular system. Thus, the wall thickness of each of the concentric tubes is in the range of from 0.025 to 0.15 mm (.001 to .006 inch). The O.D. of the outer tube 12 may then be in the range of from 0.66 to 1.47 mm (.026 to .058 inch) and that of the inner tube 20 may in the range of from 0.45 to 0.94 mm (.0177 to .037 inch).

## Claims

1. A balloon catheter comprising:
(a) a first elongated, flexible plastic tubular member (12) having a proximal end (14), a distal end (16) and describing a lumen (18) and a longitudinal axis extending from said proximal end (14) to said distal end (16);
(b) a second elongated, flexible plastic tubular member (20) having a proximal end (22), a distal end describing a lumen (26) and a longitudinal axis extending from said proximal end (22) to said distal end, said second tubular member (20) being disposed in said lumen (18) of said first tubular member (12) with a distal end portion of said second tubular member (20) extending out beyond said distal end (16) of said first tubular member (12);
(c) an inflatable expander member (30) having a proximal end (32) and a distal end (34), said proximal end (32) being circumferentially bonded to the exterior wall of said first tubular member (12) near said distal end (16) of said first tubular member (12) and said distal end (34) of said expander member (30) being circumferentially bonded to said distal end portion of said tubular member (20); and
(d) a hub member (24) affixed to said proximal end (14,22) of said first and second tubular members (12, 20) with said hub member (24) including an inflation port (26) in fluid communication with an annular space in said lumen (18) of said first tubular member (12) surrounding said second tubular member (20);
characterised in that, said balloon catheter has
(e) a wall thickness in the range of from 0.025 mm (0.001 inch) to 0.150mm (0.006 inch);
and it further comprises
(f) at least one reinforcing wire (40, 44) embedded in the wall of either or both of said first tubular member (12) or said second tubular member (20) and extending parallel to the longitudinal axis of said first and second tubular members (12, 20).

2. The catheter as in Claim 1 wherein said plastic is selected from the group of polymers including polyamide, polyimide and polyester.

3. The catheter as in Claim 1 wherein said reinforcing wire (40, 44) in one of said first and second tubular members (12, 20) includes a plurality of radially spaced wire strands (40, 44) extending parallel to the longitudinal axis of said first and second tubular members (12, 20).

4. The catheter as in any of Claims 1 through 3 wherein the outside diameter of said first tubular member (12) is in the range of from 0.66 to 1.47mm (0.26 to 0.58 inch) and the outside diameter of said second tubular member (20) is in the range of from 0.45mm to 0.94mm (0.0177 to 0.037 inch).

5. The catheter as in Claim 4 wherein said reinforcing wire (40, 44) is stainless steel wire having a diameter of 0.025mm (0.001 inch) to 0.050mm (0.002 inch).

## Patentansprüche

1. Ballonkatheter, umfassend:
(a) ein erstes langgestrecktes, flexibles Rohrglied aus Kunststoff (12) mit einem proximalen Ende (14), einem ein Lumen (18) umschreibenden distalen Ende (16) und einer Längsachse, die sich vom proximalen Ende (14) zum distalen Ende (16) erstreckt:
(b) ein zweites langgestrecktes, flexibles Rohrglied aus Kunststoff (20) mit einem proximalen Ende (22), einem ein Lumen (26) umschreibenden distalen Ende und einer sich von dem proximalen Ende (22) zum distalen Ende erstreckenden Längsachse, wobei das zweite Rohrglied (20) in dem Lumen (18) des ersten Rohrgliedes (12) angeordnet ist und ein distaler Endteil des zweiten Rohrgliedes (20) über das distale Ende (16) des ersten Rohrgliedes (12) vorsteht;
(c) ein aufpumpbares Expansionsglied (30) mit einem proximalen Ende (32) und einem distalen Ende (34), wobei das proximale Ende (32) um den Umfang herum mit der Außenwand des ersten Rohrgliedes (12) nahe dem distalen Ende (16) des ersten Rohrgliedes (12) verbunden ist und das distale Ende (34) des Expansionsgliedes (30) um den Umfang herum mit dem distalen Endteil des Rohrgliedes (20) verbunden ist; und
(d) ein Verbindungsstück (24), das am proximalen Ende (14, 22) des ersten und zweiten Rohrgliedes (12, 20) befestigt ist, wobei das Verbindungsstück (24) ein Aufpumpanschlußstück (26) in Strömungskommunikation mit einem Ringraum in dem Lumen (18) des das zweite Rohrglied (20) umgebenden ersten Rohrgliedes einschließt,
dadurch gekennzeichnet, daß der Ballonkatheter
(e) eine Wandstärke im Bereich von 0,025 mm (0,001 Inch) bis 0,150 mm (0,006 Inch) hat und er ferner
(f) mindestens einen Verstärkungsdraht (40, 44) aufweist, der in der Wand eines oder beider des ersten Rohrgliedes (12) oder des zweiten Rohrgliedes (20) eingebettet ist und parallel zur Längsache des ersten und zweiten Rohrgliedes (12, 20) verläuft.

2. Katheter nach Anspruch 1, bei dem der Kunststoff aus der Gruppe von Polymeren einschießlich Polyamid, Polyimid und Polyester ausgewählt ist.

3. Katheter nach Anspruch 1, bei dem der Verstärkungsdraht (40, 44) in einem des ersten und zweiten Rohrgliedes (12, 20) eine Vielzahl radial beabstandeter Drahtstränge (40, 44) umfaßt, die parallel zur Längsachse des ersten und zweiten Rohrgliedes (12, 20) verlaufen.

4. Katheter nach irgendeinem der Ansprüche 1 bis 3, bei dem der Außendurchmesser der ersten Rohrgliedes im Bereich von 0,66 bis 1,47 mm (0,26 bis 0,58 Inch) und der Außendurchmesser des zweiten Rohrgliedes (20) im Bereich von 0,45 bis 0,94 mm (0,0177 bis 0,037 Inch) liegen.

5. Katheter nach Anspruch 5, bei dem der Verstärkungsdraht (40, 44) ein Draht aus rostfreiem Stahl mit einem Durchmesser von 0,025 mm (0,001 Inch) bis 0,050 mm (0,002 Inch) ist.

## Revendications

1. Cathéter à ballonnet comportant :
(a) un premier élément tubulaire allongé (12) en matière plastique flexible ayant une extrémité proximale (14), une extrémité distale (16) et définissant une lumière (18) et un axe longitudinal s'étendant depuis ladite extrémité proximale (14) jusqu'à ladite extrémité distale (16) ;
(b) un second élément tubulaire allongé (20) en matière plastique flexible ayant une extrémité proximale (22), une extrémité distale définissant une lumière (26) et un axe longitudinal s'étendant depuis ladite extrémité proximale (22) jusqu'à ladite extrémité distale, ledit second élément tubulaire (20) étant disposé dans ladite lumière (18) dudit premier élément tubulaire (12) de manière qu'une partie extrême distale dudit second élément tubulaire (20) s'étende vers l'extérieur au-delà de ladite extrémité distale (16) dudit premier élément tubulaire (12) ;
(c) un élément gonflable (30) d'expansion ayant une extrémité proximale (32) et une extrémité distale (34), ladite extrémité proximale (32) étant liée circonférentiellement à la paroi extérieure dudit premier élément tubulaire (12) à proximité de ladite extrémité distale (16) dudit premier élément tubulaire (12), et ladite extrémité distale (34) dudit élément (30) d'expansion étant liée circonférentiellement à ladite partie extrême distale dudit élément tubulaire (20) ; et
(d) un élément central (24) fixé ladite extrémité proximale (14, 22) desdits premier et second éléments tubulaires (12, 20), ledit élément central (24) présentant un orifice (26) de gonflage en communication de fluide avec un espace annulaire dans ladite lumière (18) dudit premier élément tubulaire (12) entourant ledit second élément tubulaire (20) ;
caractérisé en ce que ledit cathéter à ballonnet présente
(e) une épaisseur de paroi dans la plage de 0,025 mm (0,001 inch) à 0,150 mm (0,006 inch);
et en ce qu'il comporte en outre
(f) au moins un fil métallique (40, 44) de renfort noyé dans la paroi de l'un desdits premier élément tubulaire (12) et second élément tubulaire (20) ou des deux et s'étendant parallèlement à l'axe longitudinal desdits premier et second éléments tubulaires (12, 20).

2. Cathéter selon la revendication 1, dans lequel ladite matière plastique est choisie dans le groupe de polymères comprenant un polyamide, un polyimide et un polyester.

3. Cathéter selon la revendication 1, dans lequel ledit fil métallique (40, 44) de renfort dans l'un desdits premier et second éléments tubulaires (12, 20) comprend plusieurs brins (40, 44) de fil métallique espacés radialement et s'étendant parallèlement à l'axe longitudinal desdits premier et second éléments tubulaires (12, 20).

4. Cathéter selon l'une quelconque des revendications 1 à 3, dans lequel le diamètre extérieur dudit premier élément tubulaire (12) est dans la plage de 0,66 à 1,47 mm (0,26 à 0,58 inch) et le diamètre extérieur dudit second élément tubulaire (20) est dans la plage de 0,45 mm à 0,94 mm (0,0177 à 0,037 inch).

5. Cathéter selon la revendication 5, dans lequel ledit fil métallique (40, 44) de renfort est un fil d'acier inoxydable ayant un diamètre de 0,025 mm (0,001 inch) à 0,050 mm (0,002 inch).
